# EUROPEAN PATENT SPECIFICATION

(11) **EP 0 400 695 B1**
(45) Date of publication and mention of the grant of the patent: **12.07.1995**
(21) Application number: 90200513.1
(22) Date of filing: 05.03.1990
(51) Int. Cl.: C07J 41/00

(54) **A process for the preparation of tauroursodesoxycholic acid dihydrate**
Verfahren zur Herstellung von Tauroursodesoxycholsäure Dihydrat
Procédé pour préparer l'acide tauroursodésoxycholique dihydraté

(30) Priority: 30.05.1989 IT 2069889
(43) Date of publication of application: 05.12.1990
(73) Proprietor: PRODOTTI CHIMICI E ALIMENTARI SPA, 15060 Basaluzzo (Alessandria) (IT)
(72) Inventor: Parenti, Massimo, I-15060 Novi Ligure (Alessandria) (IT)
(74) Representative: Michelotti, Giuliano

(56) References cited:
- EP-A- 0 119 040
- EP-A- 0 272 462
- BE-A- 901 069

## Description

The present invention relates to the preparation of dihydrated tauroursodesoxycolic acid and more particularly to a process for the purification of said acid as obtained in raw form from the reaction between taurine and the mixed anhydride of the ursodesoxycolic acid.

In the preparation of this acid, the therapeutical activity of which in the treatment of biliar diseases and misfunctions is known, according to the Italian Patent No. 1.197.330 of the same Applicant, taurine, in form of aqueous solution of an alkali salt thereof, is reacted with the mixed anhydride of ursodesoxycholic acid.

The latter is prepared through the reaction between ursodesoxycholic acid and an alkyl chloroformiate, particularly methyl or ethyl-chloroformiate, in the presence of a tertiary base, such as triethylamine.

The reaction takes place with high yields and in an industrially advantageous manner, but the reaction raw product contains several by-products of reaction besides obviously unreacted reactants (taurine and ursodesoxycholic acid).

Among the by-products the following are included:
- the tertiary amine;
- chlorides originated from the hydrolysis of the unreacted alkylchloroformiate;
Of course for a therapeutical use of the said compound these impurities must be removed in a substantial degree and without prejudice of the yields.

In the case of the sodium salt of the coniugates with taurine and glycine of the chenodesoxycholic acid (Hofmann A.F., Acta Chem. Scan. 17 (1963), 173-186) is it known to carry out the purification from the tertiary amine by means of a cationic resin, different steps and methods being however foreseen for the remowal of the other impurities (by-products and unreacted reactants), particularly steps involving solvent extractions and repeated crystallization.

In the case of the tauroursodesoxycholic acid the product of the conjugation is in fact the sodium salt of the said acid from which, according to the standard method of the chemistry, the acid is released by merely adding a suitable acid, for example and preferably hydrochloric acid.

Consequently salts, particularly chlorides, are still formed which must be removed as impurities.

Even having recourse to subsequent crystallization, there remain in the product not negligible percentages of impurities, which, in view of the therapeutical use, make it necessary further time-consuming and expensive purification steps, which obviously are fully not consistent with a production on industrial scale.

The purpose of the present invention is thus that of solving the problem as above shortly mentioned in an industrially acceptable and advantageous manner.

It has been now surprisingly found and is the object of the present invention that if the aqueous solution resulting from the reaction between the mixed anhydride of the ursodesoxycholic acid and the taurine is serially percolated through a first strong cation exchange resin and through a second weak anionic exchange resin, the raw reaction product is substantially depurated from the tertiary amine base and from the chlorides, whereby the purification in a known manner from unreacted taurine and from ursodesoxycolic acid is readily and easily carried out, leading to the dihydrated tauroursodesoxycholic acid in pure form suitable for the pharmaceutical use.

It has been particularly found and this is one of the advantages of the present invention, that the passage through the first column can be carried out in a simple and safe manner since the raw reaction solution, containing the sodium salt of tauroursodesoxycholic acid, has a pH from neutral to weakly alkaline, whereas the free acid is a strong acid and the aqueous solution thereof has a definitely acid pH (2-3). Consequently by monitoring the eluate of the first column and particularly the pH thereof or a parameter related thereto, it is easy to operatingly individuate the beginning and the end of the phase in which the acid can be released and consequently in the eluate substantially pure acid passes (apart from unreacted taurine and ursodesoxycolic acid), whereby more timeconsuming and mainly more complicated analyses are no longer necessary.

Secondly it is no longer necessary to add a mineral acid, particularly hydrochloric acid, to the raw reaction product to recover as already indicated the free acid, whereby not only further impurities are avoided, but also losses of the desired active principle, namely even modest reductions of yields are avoided.

A further advantage, which is readily appreciated by referring for example to the above mentioned paper by Hofmann, is the fact the purification is simple and reliable, which, in the production of a therapeutically active principle, has remarkable importance.

Obviously the selection of the ion exchange resin shall not be difficult for the skilled man, provided that the cationic resin is strong, in H⁺ form, whereas the anionic one is of weak type.

The following example illustrates, without any limiting meaning, the purification process according to the invention.

### EXAMPLE

### a-) Mixed anhydride of the ursodesoxycholic acid.

A solution of 48 g of ursodesoxycholic acid and 12.5 g of triethylamine in 300 ml of dioxane, cooled to -5°C, is added with 13.4 g of ethylchloroformiate.

The reaction is exothermal and the internal temperature of -5°C is maintained by externally cooling by means of acetone and dry ice. Upon the addition is complete, the mixture is maintained under stirring for 15-30 minutes, leaving the suspension to heat up to the room temperature.

The suspension is filtered from triethylamine hydrochloride and the filtered organic solution is used for the next reaction.

### b) Sodium salt of tauroursodesoxycolic acid.

A solution of 19.2 g of taurine in 120 ml of 1N sodium hydroxide is prepared.

This solution is poured onto the solution of mixed anhydride prepared according to the above example.

The reaction is stirred for 3 hours and after that time it is filtered to remove the unreacted taurine.

### c) Dihydrated tauroursodesoxycholic acid.

The solution of sodium salt of the tauroursodesoxycholic acid of the above example (500 ml) is percolated through a column packed with 100 ml of strong cationic resin, Relite CF, in ionic form H⁺, at a rate of 500 ml/hour.

The solution of dihydrated tauroursodesoxycholic acid percolated from the Relite CF resin is percolated again on 100 ml of Relite GH 1 resin, of weak anionic type, in the ionic form of a free base at the rate of 500 ml/hour. The percolated solution is concentrated to dryness under vacuum, keeping the temperature not above 50°C. 120 ml of ethanol are added and the mixture is refluxed for 1 hour.

The filtered solution is precipitated by adding 3 lt of acetone cooled at 0°C.

After a night in the refrigerator the mixture is filtered and dried at 50°C under vacuum, giving 50 g of tauroursodesoxicholic acid.

This acid is dissolved in 150 ml of deionized water and precipitated with 3 lt of cool acetone (0°C).

After one night in refrigerator the acid is filtered and dried under vacuum at 50°C, giving 20 g of dihydrated tauroursodesoxycholic acid having a purity of 99.5% and melting point, with decomposition, of 143°C.

## Claims

1. A process for the purification of dihydrated tauroursodesoxycholic acid, obtained through the reaction between taurine, in form of aqueous solution of an alkali salt thereof, and the mixed anhydride of the ursodesoxycholic acid with an alkyl chloroformiate, characterized in that the raw raction product is passed serially through a first column of strong cationic exchange resin and through a second column of weak anionic exhange resin, the purification in a per se known manner from unreacted taurine and ursodesoxycholic acid being thereafter carried out.

2. A process according to claim 1, characterized in that said strong cationic exhange resin is a cationic resin in form H⁺.

3. A process according to claim 1, characterized in that the pH of the eluate of said first column or a factor related thereto is monitored, removing the fraction corresponding to the definite variation of pH from a value of between neutral and weakly alkaline and a definitely acid value.

## Patentansprüche

1. Verfahren zur Reinigung von dihydratisierter Tauroursodesoxycholsäure, erhalten durch Reaktion zwischen Taurin in Form einer wäßrigen Lösung eines Alkalisalzes davon und dem gemischten Anydrid von Ursodesoxycholsäure mit einem Chlorameisensäurealkylester, dadurch gekennzeichnet, daß das rohe Reaktionsprodukt nacheinander durch eine erste Säule eines stark kationischen Austauscherharzes und durch eine zweite Säule eines schwach anionischen Austauscherharzes geleitet wird und danach die Reinigung von nicht-reagiertem Taurin und Ursodesoxycholsäure in an sich bekannter Weise durchgeführt wird.

2. Verfahren nach Anspruch 1, dadurch gekennzeichnet, daß das stark kationische Austauscherharz ein kationisches Harz in der H⁺ Form ist.

3. Verfahren nach Anspruch 1, dadurch gekennzeichnet, daß der pH des Eluats der ersten Säule oder ein damit in Beziehung stehender Faktor überwacht wird, wobei die Fraktion entfernt wird, die einer deutlichen pH-Änderung von einem Wert zwischen neutral und schwach alkalisch und einem deutlich sauren Wert entspricht.

## Revendications

1. Un procédé pour la purification de l'acide tauroursodésoxycholique dihydraté, obtenu par la réaction de la taurine, sous forme d'une solution aqueuse de sels alcalins sur l'anhydride mixte de l'acide ursodésoxycholique avec un chloroformiate d'alcoyle, caractérisé en ce que le produit brut est passé successivement sur une première colonne de résine échangeuse fortement cationique et sur une deuxième colonne de résine échangeuse faiblement anionique, la purification étant ensuite conduite d'une manière en soi connue pour éliminer la taurine et l'acide oursodésoxychlolique n'ayant pas réagi.

2. Un procédé selon la revendication 1, caractérisé en ce que ladite résine échangeuse de cation fort est une résine cationique sous sa forme H⁺.

3. Un procédé selon la revendication 1, caractérisé en ce que le pH de l'éluat sortant de ladite première colonne ou le facteur qui y est lié, est contrôlé, pour retirer la fraction correspondant à une gamme définie de pH comprise entre la neutralité et une valeur faiblement alcaline déterminée, d'une part et une valeur acide définie, d'autre part.
